# EUROPÄISCHE PATENTANMELDUNG

(11) **EP 3 766 340 A1**
(43) Veröffentlichungstag der Anmeldung: **20.01.2021**
(21) Anmeldenummer: 19187371.0
(22) Anmeldetag: 19.07.2019
(51) Int. Cl.: A01K 5/01, A01K 13/00, A01K 29/00, A61L 2/10, A01K 1/00, A01K 11/00

(54) **BEHANDLUNGSSTATION UND VERFAHREN ZUR INSBESONDERE AUTOMATISIERTEN KEIMREDUZIERENDEN BESTRAHLUNG DES INTEGUMENTS VON LANDWIRTSCHAFTLICHEN NUTZTIEREN, INSBESONDERE VON JUNGTIEREN, Z. B. KÄLBERN, UND ALS EINE DERARTIGE BEHANDLUNGSSTATION AUSGEBILDETE TRÄNKESTATION SOWIE FÜTTERUNGSAUTOMAT MIT EINER DERARTIGEN TRÄNKESTATION**

(71) Anmelder: Urban Holding GmbH, 27798 Hude/Wüsting (DE)
(72) Erfinder: Sprock, Thomas, 27798 Hude-Wüsting (DE)
(74) Vertreter: Manasse, Uwe

(57) **Zusammenfassung**

Behandlungsstation und Verfahren zur insbesondere automatisierten keimreduzierenden Bestrahlung des Integuments von Tieren, insbesondere von Jungtieren, z. B. Kälbern, wobei die Behandlungsstation umfasst: eine Tier-Einzelbox, einen Tier-Einzelunterstand oder dergleichen für den Innen- und/oder Außenbereich, umfassend mehrere Seitenwände, ein Dach und einen offenen Zugang, und mindestens eine UV-C-Keimreduzierungseinrichtung zur keimreduzierenden Bestrahlung des Integuments von einem in der Tier-Einzelbox bzw. in dem Tier-Einzelunterstand oder dergleichen befindlichen landwirtschaftlichen Nutztier, insbesondere Jungtier, z. B. Kalb, eine als eine derartige Behandlungsstation ausgebildete Tränkestation, ein Fütterungsautomat mit derselben sowie ein System damit.

## Beschreibung

Die vorliegende Erfindung betrifft eine Behandlungsstation und ein Verfahren zur insbesondere automatisierten keimreduzierenden Bestrahlung des Integuments von landwirtschaftlichen Nutztieren, insbesondere von Jungtieren, z. B. von Kälbern.

Die vorliegende Erfindung betrifft das technische Gebiet der landwirtschaftlichen Nutztierhaltung, speziell die Haltung von Jungtieren.

Zu den kennzeichnenden Merkmalen von Säugetieren gehört das Säugen des Nachwuchses mit Milch. Hierbei saugen die Jungtiere zum Zwecke der Aufnahme von Muttermilch am Gesäuge des Muttertieres oder - im Falle der landwirtschaftlichen Nutztierhaltung - an einer dem Gesäuge des Muttertieres nachempfundenen Kunstzitze. Die Nahrungsverabreichung an dieser Zitze, oft als Nuckel oder Sauger tituliert, geht vielmals automatisiert vonstatten, wobei für die Zubereitung und Abgabe des Nahrungsmittels, meist Milchtränke, Fütterungsautomaten zum Einsatz kommen.

Gerade beim Einsatz solcher Fütterungsautomaten sind die landwirtschaftlich gehaltenen Jungtiere, oftmals Kälber, meist in einer Gruppe zusammen mit mehreren anderen Jungtieren aufgestallt, wobei die gesamte Tiergruppe ihre Nahrung dann an einem der Gruppe zugeordneten Tränkestand aufnimmt. Diese Haltungsform wird als Gruppenhaltung bezeichnet. Sie ist für Kälber ab einem Alter von 8 Wochen gesetzlich vorgeschrieben (Tierschutz-Nutztierhaltungsverordnung, §8). Die Gruppenhaltung ist nun insofern problematisch, als dass die Tiere durch Körperkontakt untereinander auch Krankheiten und Krankheitskeime auf ihre Artgenossen (und auch auf den Tierbetreuer) übertragen können. Die feucht-warme Stallumgebung stellt einen idealen Nährboden für die Entwicklung von Keimen und Bakterien dar. Insofern besteht im Stall die Gefahr, dass im Zuge von Sozialkontakten mit Artgenossen oder durch den Kontakt mit der Buchteneinrichtung unerwünschter Weise Krankheitserreger von einem Tier auf das nächste Tier übertragen werden. Über diese sog. Schmierinfektionen kann es leicht zu einer sog. Kreuzkontamination kommen, wodurch sich bei virulenten Erregern mehr oder minder schnell viele Tiere der Gruppe infizieren und in der Folge erkranken können.

Eine bei Kälbern und Jungrindern häufig auftretende Krankheit ist die Rindertrichophytie, auch als sog. Kälberflechte oder Glatzflechte betitelt. Hierbei handelt es sich um eine Dermatophytose, welche auch auf den Menschen übertragbar ist. Verursacher sind Hautpilze der Gattung Trichopython, insbesondere die Spezies *Trichopython υerrucosum.* Diese in den oberen Hautschichten lebenden Pilze dringen in die Hautfollikel ein und schädigen diese. Es entstehen meist rundliche Entzündungsherde, welche nässen oder sogar bluten können. Meist kommt es zu Sekundärinfektionen, im weiteren Verlauf fallen befallenen Tieren lokal die Haare aus und es bilden sich borkige-schuppige Krusten, welche mit Juckreiz verbunden sind. Bei betroffenen Tieren kommt es zu Unruhe und verminderter Futteraufnahme, die Entwicklung der Jungtiere wird verzögert, da viel Energie für die Immunabwehr mobilisiert werden muss. Betroffene Tiere haben einen geringeren Immunstatus und sind somit anfälliger gegen andere Krankheiten. Nicht zuletzt beeinträchtigt die Erkrankung auch die spätere Lederqualität. Schätzungen gehen davon aus, dass in Deutschland 40% der Bestände mit *Trichopython verrucosum* infiziert sind. Die Sporen des Pilzes können in trockener Umgebung über mehrere Jahre infektiös bleiben, was die Behandlung erschwert. Zwar können betroffene Tiere medikamentös behandelt werden, um jedoch langfristig eine Verbesserung der Flechtenproblematik zu erreichen, ist die zweimalige Impfung des gesamten Jungviehbestandes und aller neu dazukommenden Kälber mit Lebendimpfstoffen über mehrere Monate bis Jahre nötig.

Gerade im Bereich der Fresseraufzucht stellt Trichopythie oftmals ein Problem dar, da fresserhaltende Betriebe ihre aufzuziehenden Tiere in regelmäßigem Turnus aus mehreren verschiedenen Betrieben zukaufen und nach erfolgter Aufzucht dann wieder an Bullenmastbetriebe abgeben. In solch einer Konstellation können lediglich kurz- oder allenfalls mittelfristige realisierbare Behandlungsstrategien umgesetzt werden.

Während die Rinderflechte auch bei älteren Tieren stark ausgeprägt ist, treten Durchfallerkrankungen vornehmlich bei sehr jungen Kälbern auf.

Durchfallerkrankungen können fütterungs- und hygienebedingte Ursachen haben, oftmals ist die Ursache der Erkrankung eine Ansteckung mit Krankheitserregern. Sind diese Erreger infektiös, kann schnell ein Großteil der Tiergruppe betroffen sein.

Häufig anzutreffende, Durchfall verursachende Krankheitserreger bei Kälbern sind zum einen Viren, wie etwa Rota- und/oder Coronaviren. Als weitere Erreger von Kälberdurchfällen sind Kryptosporidien, eine Gattung einzelliger Parasiten, zu benennen. Speziell die Spezies *Cryptosporidium parvum* verursacht Durchfälle bei Kälbern, sehr oft bereits in den ersten beiden Lebenswochen. Von Durchfall betroffene Kälber weisen massive gesundheitliche Probleme auf, in deren Folge es zu erheblichen wirtschaftlichen Schäden für den Tierhalter kommt.

Kryptosporidien kommen, wie sehr viele andere Endoparasiten auch, in verschiedenen Entwicklungsstadien vor und haben einen komplexen Lebenszyklus.

Kryptosporidien bilden als Dauerstadien sehr widerstandsfähige Oocysten aus, die hochinfektiös sind und dies über Monate hinweg bleiben. In einem Gramm Kot eines infizierten Tieres können sich bis zu 5 Millionen Oocysten befinden.

Bereits die orale Aufnahme von weniger als 100 Oocysten kann zur Infektion eines Kalbes führen. Diese Oocysten können aufgenommen werden etwa durch das Abschlucken von Einstreu, welche Kotpartikel mit Oocysten enthält. Oftmals erfolgt die orale Infektion der Tiere mit Oocysten aber auch dadurch, dass die Tiere das Fell eines Artgenossen belecken und in diesem Zuge Oocysten abschlucken. Solche Sozialkontakte sind Teil des natürlichen Verhaltensrepertoires eines Kalbes und werden von den Tieren regelmäßig praktiziert. Bei Kälberdurchfällen sind der Analbereich, das umliegende Fell und oft auch die Hinterbeine betroffener Tiere meist mehr oder weniger mit Kot verschmutzt, oft sind auch Buchtenwände oder Einrichtungsgegenstände mit infektiösem Kot beschmiert. Insofern sind hier sog. Schmierinfektionen, also die Übertragung der Erreger durch Berührung eines Artgenossen oder durch Kontakt mit der Buchteneinrichtung oder der Einstreu ein häufig auftretender Auslöser für Neuinfektionen.

Nach einer erfolgten Infektion (Abschlucken von Oocysten) vermehren sich die Kryptosporidien im Verdauungstrakt des Tieres sehr schnell und effektiv. Die gebildeten Oocysten werden dann wieder mit dem Kot ausgeschieden und können weitere Tiere infizieren.

Die Behandlung einer Kryptosporidiose ist teuer und ineffektiv. Es gibt kaum Medikamente zur Bekämpfung dieser Krankheit und die einzigen vorliegenden Arzneimittel sind mit starken Nebenwirkungen verbunden. Insofern ist und bleibt das wirksamste Mittel zur Verhinderung und/oder Bekämpfung der Krankheit das Schaffen und Aufrechterhalten einer hygienischen Haltungsumgebung.

Cryptosporidium-Oocysten verfügen über eine sehr stabile Hülle. Sie sind sehr langlebig und können Wochen und Monate überdauern. Mittels einer herkömmlichen Stallreinigung (bloßes Ausspritzen der Abteile mit einem Hochdruckreiniger) sind Kryptosporidien nicht wirksam zu bekämpfen. Der Einsatz von Desinfektionsmitteln ist obligat für eine wirksame Bekämpfung und Eindämmung von durch Kryptosporidien verursachten Neugeboreneninfektionen.

Desinfektionsmittel sind jedoch vergleichsweise teure Verbrauchsmittel, die immer nur spezifisch gegen einen oder mehrere bestimmte Erreger wirksam sind. Ein universelles, alle Keime und Bakterien abtötendes Breitband-Desinfektionsmittel gibt es nicht. Da die Oocysten aufgrund ihrer sehr stabilen Hülle unempfindlich gegen herkömmliche Desinfektionsmittel auf Chlorid-Basis sind, müssen sie mit speziellen Desinfektionsmitteln behandelt werden. Diese Behandlung ist teuer und zeitaufwändig, erfordert sie doch mehrere Schritte: Grobreinigung des Stalls mit einem Hochdruckreiniger, Ausbringen und Einwirken des Desinfektionsmittelns, Ausspritzen und anschließendes Austrocknen des Stalls.

Ferner ist der Einsatz von Desinfektionsmitteln insofern problematisch, als dass es sich bei den eingesetzten Stoffen durchweg um Biozide handelt, die potentiell sowohl für den Anwender als auch für die Umwelt schädlich sind, was entsprechende Schutzmaßnahmen erforderlich macht, die auf landwirtschaftlichen Betrieben nicht immer eingehalten werden können. Zudem besteht die Gefahr, dass die zu bekämpfenden Mikroorganismen früher oder später Resistenzen gegen die Wirkstoffe der Desinfektionsmittel ausbilden, welche deren Wirksamkeit einschränken oder diese im Lauf der Zeit gänzlich unwirksam werden lassen.

Die Problematik der Keimübertragung und -verschleppung von Tier zu Tier ist wohlbekannt und stellt insbesondere bei größeren Stalleinheiten ein Problem dar. Gängige Gegenmaßnahmen sind die Anwendung des sog. Rein-Raus-Verfahrens bei der Belegung der Stalleinheiten und die routinemäßige Reinigung der Abteile vor der Neubelegung, bei Bedarf zusätzlich flankierend begleitet von der Desinfektion derselben. Diese Maßnahmen zielen stark auf eine Hygienisierung der Haltungsumwelt ab. Die Keimübertragung von Tier zu Tier wird nicht adressiert.

Der vorliegenden Erfindung liegt nun die Aufgabe zugrunde, die Übertragung von Keimen und Bakterien ausgehend von der Felloberfläche der Tiere stark zu reduzieren, insbesondere im Hinblick auf Kryptosporidien-Oocysten und im Hinblick auf Pilze und oder Sporen der Gattung Trichopython.

Erfindungsgemäß wird diese Aufgabe gemäß einem ersten Aspekt gelöst durch eine Behandlungsstation zur insbesondere automatisierten keimreduzierenden Bestrahlung des Integuments von landwirtschaftlichen Nutztieren, insbesondere von Jungtieren, z. B. Kälbern, umfassend:
eine Tier-Einzelbox, einen Tier-Einzelunterstand oder dergleichen für den Innen- und/oder Außenbereich, umfassend mehrere Seitenwände, ein Dach und einen offenen Zugang, und
mindestens eine UV-C-Keimreduzierungseinrichtung zur keimreduzierenden Bestrahlung des Integuments von einem in der Tier-Einzelbox bzw. in dem Tier-Einzelunterstand oder dergleichen befindlichen landwirtschaftlichen Nutztier, insbesondere Jungtier, z. B. Kalb.

Der Begriff "Seitenwand" soll auch Vorder- und Rückwände umfassen.

Weiterhin wird diese Aufgabe gemäß einem weiteren Aspekt gelöst durch eine als eine Behandlungsstation nach einem der Ansprüche 1 bis 9 ausgebildete Tränkestation, umfassend eine Entnahmeeinrichtung zum Entnehmen eines flüssigen Futtermittels, insbesondere Milchtränke, aus einem Fütterungsautomaten oder zum Entnehmen von Tränkewasser.

Weiterhin liefert die vorliegende Erfindung gemäß einem weiteren Aspekt einen Fütterungsautomaten, umfassend mindestens eine Tränkestation nach einem der Ansprüche 10 bis 13.

Darüber hinaus wird diese Aufgabe gemäß einem weiteren Aspekt gelöst durch ein System aus einer Behandlungsstation nach einem der Ansprüche 1 bis 9, einer Tränkestation nach einem der Ansprüche 10 bis 13 oder einem Fütterungsautomaten und mindestens einer Schutzausrüstung zum Schutz eines Tieres vor UV-C-Strahlung, wobei die Schutzausrüstung mindestens eine Schutzbrille oder Schutzkontaktlinsen umfasst.

Zudem wird diese Aufgabe gemäß einem weiteren Aspekt gelöst durch ein Verfahren zur insbesondere automatisierten keimreduzierenden Bestrahlung des Integuments von landwirtschaftlichen Nutztieren, insbesondere von Jungtieren, z. B. von Kälbern, vorzugsweise unter Verwendung einer Behandlungsstation nach einem der Ansprüche 1 bis 9 oder einer Tränkestation nach einem der Ansprüche 10 bis 14 oder eines Systems nach Anspruch 15, umfassend die Schritte:
- Automatisierte Identifikation eines Tieres, Ermittlung der Notwendigkeit einer Behandlung des Tieres mit UV-C-Strahlung und Abruf einer vorab tierindividuell festgelegten Behandlungsdauer anhand eines Tierkennungs-Chips in der Behandlungsstation oder Tränkestation und
- Behandlung des Tieres mit UV-C-Strahlung mit besagter Behandlungsdauer.

Zum Beispiel können ein oder mehrere Tiere in einem Gruppenstall vorab zur Behandlung markiert werden und kann eine tierindividuelle Festlegung der Behandlungsdauer erfolgen.

Bei der Behandlungsstation kann vorgesehen sein, dass die UV-C-Keimreduzierungseinrichtung mindestens einen UV-C-Strahler umfasst.

Vorteilhafterweise ist der UV-C-Strahler mittels eines Akkus und/oder einer Batterie betreibbar. Selbstverständlich ist aber auch ein Netzbetrieb denkbar.

Vorteilhafterweise umfasst die Behandlungsstation ferner eine Reinigungseinrichtung für den UV-C-Strahler.

Gemäß einer besonderen Ausführungsform der Behandlungsstation ist die UV-C-Keimreduzierungseinrichtung in mindestens einer der Seitenwände und/oder dem Dach integriert.

Vorteilhafterweise umfasst die Behandlungsstation ferner eine Tieridentifikationseinrichtung.

Zweckmäßigerweise ist der offene Zugang nach Betreten eines Tieres automatisiert verschließbar.

Gemäß einer weiteren besonderen Ausführungsform der vorliegenden Erfindung weist die Behandlungsstation ferner eine Steuereinrichtung zur Einschaltung der UV-C-Keimreduzierungseinrichtung für eine vorgebbare, vorzugsweise tierindividuelle, Zeitdauer, beginnend bei oder nach Betreten eines Tieres.

Gemäß einer weiteren besonderen Ausführungsform umfasst die Behandlungsstation ferner eine Anwesenheits- oder Annäherungsdetektionseinrichtung zum Detektieren der Anwesenheit eines Tieres in der Tier-Einzelbox oder dem Tier-Einzelunterstand oder dergleichen oder der Annährung eines Tieres an die Tier-Einzelbox oder dem Tier-Einzelunterstand oder dergleichen.

Vorteilhafterweise ist bei der Tränkestation die Entnahmeeinrichtung außerhalb der Tier-Einzelbox bzw. des Tier-Einzelunterstandes oder dergleichen angeordnet und durch eine Öffnung in einer der Seitenwände für ein in der Tier-Einzelbox bzw. dem Tier-Einzelunterstand befindliches Tier von innen zugänglich.

Günstigerweise umfasst die Tränkestation eine Trinkdetektionseinrichtung, um Trinken eines in der Tränkestation befindlichen Tieres detektieren zu können. Beispielsweise kann ein Saugsensor vorgesehen sein, mit dem feststellbar ist, ob ein Tier derzeit beispielsweise an einem Nuckel saugt und beispielsweise die Bestrahlung des Tieres gestoppt wird, sobald das Tier seine Nahrungsaufnahme temporär unterbricht oder beendet.

Gemäß einer besonderen Ausführungsform der Tränkevorrichtung umfasst diese ferner eine Tränkestationselektronik, die gestaltet ist, um in Abhängigkeit von einer Detektion eines trinkenden Tieres mittels der Trinkdetektionseinrichtung die mindestens eine UV-C-Keimreduzierungseinrichtung ein- und auszuschalten.

Schließlich kann bei dem Verfahren vorgesehen sein, dass es ferner eine Dokumentation eines Bestrahlungsergebnisses umfasst. Beispielsweise kann der Bestrahlungserfolg dokumentiert werden.

Die vorliegende Erfindung setzt grundsätzlich auf die Nutzung von UV-C-Strahlung zur Keimreduzierung am Integument des Tieres. Sie macht sich dabei die Tatsache zunutze, dass sich Mikroorganismen gut durch Bestrahlung mit UV-C Licht abtöten lassen. Eine solche Bestrahlung ist sowohl geeignet für die Abtötung von Pilzen und/oder Sporen der Gattung Trichopython als auch für die Inaktivierung von Kryptosporidien-Oocysten.

Die Bestrahlung kann in einer Behandlungsstation mit und ohne Einrichtungen zur Aufnahme von insbesondere flüssiger Nahrung erfolgen.

Die vorliegende Erfindung macht sich nun darüber hinaus gemäß einem Aspekt die Tatsache zunutze, dass von einem Fütterungsautomaten versorgte Kälber mehrmals am Tag eine Tränkestation zur Nahrungsaufnahme freiwillig aufsuchen. In diesem Zuge kann potentiell über einen längeren Zeitraum hin eine tägliche Bestrahlung des Tieres erfolgen. Die Breite der Tränkestation ist vorzugsweise kaum größer als die Tierbreite, um ein Bedrängen eines Tieres in der Tränkestation durch Artgenossen zu verhindern. Insofern werden sich die in den Wänden und/oder der Deckplatte der Tränkestation befindlichen Strahlenquellen sehr nahe am Tierkörper befinden. Solch eine Anordnung ist effektiv und ermöglicht nur sehr kurze Bestrahlungsdauern z. B. im Minutenbereich. Da die UV-C-Strahlung mit dem Quadrat der Entfernung abnimmt, sind bereits bei geringfügig höheren Abständen zwischen Tierfell und Strahlenquelle wesentlich längere Bestrahlungsdauern erforderlich.

Bei Tränkestationen für Kälber ist der Nuckel in der Regel außerhalb der Tränkestation angeordnet. Dies erfolgt primär unter dem Aspekt, dass der beim Saugen am Nuckel abgegebene Speichel nicht auf den Boden innerhalb der Tränkestation tropfen soll. Bei solch einer Anordnung befinden sich der Kopf und die besonders empfindlichen Augen des Tieres beim Saugen außerhalb der Tränkestation und sind nicht der UV-C Strahlung ausgesetzt. Die Bestrahlung in der Tränkestation findet vorteilhafterweise erst dann statt, wenn z. B. ein Kalb tatsächlich Milch trinkt, was z. B. durch einen Durchflusssensor in der Saugleitung sehr einfach detektiert werden kann. Über diesen Durchflusssensor können gemäß einer besonderen Ausführungsform auch Unterbrechungen oder die Beendigung des Saugvorgangs detektiert werden, woraufhin die UV-C-Strahler dann temporär oder permanent ausgeschaltet werden. Innerhalb einer Tränkestation können die UV-C-Strahler gut gegen Staub geschützt werden.

Bei älteren Jungtieren, welche von der Milch abgesetzt wurden und welche daher nicht mehr an einer Milchtränkestation versorgt werden, kann die Bestrahlung in gleicher Weise in einer Wassertränkestation vorgenommen werden, auch diese Station wird von den Tieren mehrmals täglich frequentiert.

Sowohl Tränkestationen für die Aufnahme von Milchtränke (Milchtränkestationen) als auch Tränkestationen zur Wasseraufnahme (Wassertränkestationen) können z. B. konstruktiv so gestaltet werden, dass sich der Kopf des Tieres während der Milchtränke- bzw. Wasseraufnahme außerhalb der Tränkestation (Milch- bzw. Wassertränkestation) befindet und damit nicht der Strahlung ausgesetzt ist. Dies ist insofern angebracht, als gerade die Augen sehr stark strahlungsgefährdet sind, desgleichen auch die nicht von Fell bedeckten Hautpartien im Kopfbereich (Flotzmaul). Andererseits ist bei Rindertrichopythie gerade oftmals auch der Kopfbereich der Tiere sehr stark betroffen, weswegen eine Bestrahlung des Kopfbereichs durchaus angebracht und sinnvoll sein kann. In solch einem Fall werden vorteilhafterweise die empfindlichen Augen der Tiere durch eine UV-Schutzbrille geschützt, um das Eindringen der UV-C Strahlen auf die Hornhaut und in die Augen der Tiere zu verhindern. Gleicherweise sind auch käuflich erwerbbare Schutz-Kontaktlinsen für Tiere dazu geeignet, die Augen des Tieres vor der UV-C Strahlung zu schützen.

Ferner ist die Erfindung dazu geeignet, eine vorliegende Rindertrichophytie ohne Impfung und ohne medikamentöse Behandlung der Haut- und Felloberfläche direkt zu behandeln.

Bei UV-Licht handelt es sich um elektromagnetische Strahlung mit einer Wellenlänge im Bereich von 100 nm bis 390 nm. Die Wellenlänge dieses Lichts liegt außerhalb des vom menschlichen Auge sichtbaren Spektralbereichs, kann aber etwa von Bienen durchaus wahrgenommen werden.

UV-Licht selbst wird in Abhängigkeit von der Wellenlänge wiederum in drei Kategorien eingeteilt: UV-A-Strahlung (Wellenlänge von 380 nm bis 315 nm), UV-B Strahlung (315 nm bis 280 nm) und UV-C-Strahlung (280 nm bis 100 nm).

UV-A- und UV-B-Strahlung dringen beide auf die Erde durch. Insbesondere UV-B-Strahlung wird vom Körper für die Vitamin D3-Bildung benötigt. Bei übermäßiger Exposition besteht jedoch die Gefahr von Sonnenbrand. Bei beständiger, langanhaltender Exposition steigt auch das Risiko an, an Hautkrebs zu erkranken.

Demgegenüber wird die UV-C-Strahlung von den obersten Luftschichten der Erdatmosphäre absorbiert, selbst im Bereich des Ozonlochs.

Die UV-C-Strahlung ist die energiereichste der drei UV-Strahlungstypen. Sie dringt zwar kaum in die Haut ein, schädigt jedoch massiv das Erbmaterial und wirkt daher stark keimreduzierend.

Die massive Schädigung des Erbmaterials (DNA) durch UV-C-Licht beruht darauf, dass die Photonen der UV-C-Strahlung eine Brückenbildung von zwei nebeneinander auf einem DNA-Helixstrang liegenden Thymin-Basen bewirken können. Durch die Ausbildung dieser sog. Thymindimere wird die Replikation und Transkription der DNA effektiv unterbunden. Eine durch UV-C-Bestrahlung derart veränderte Zelle kann sich nicht mehr teilen und keine neuen Proteine mehr herstellen. Sie stirbt ab. Die vorliegende Erfindung setzt zum Zweck der Keimreduzierung gänzlich oder in Kombination mit anderen Maßnahmen auf den Einsatz von UV-C-Strahlung gemäß dem oben beschriebenen Wirkmechanismus.

Die höchste Wirksamkeit bezüglich der Keimreduzierung wird bei Bestrahlung mit UV-Licht in einem Spektralbereich von 250 nm bis 270 nm erzielt, da in diesem Wellenbereich die Absorption der Strahlung durch die DNA am höchsten ist. Insofern sind für die Keimreduzierung auf dem Integument der Tiere vorzugsweise spezifische UV-C-Strahler zu verwenden, deren Emissionscharakteristik auf diese Anforderungen hin angepasst ist.

Im Vergleich zum bereits beschriebenen Alternativverfahren der Keimreduzierung mittels Desinfektionsmitteln weist der Einsatz von UV-C-Strahlung zu diesem Zweck wesentliche Vorteile auf: die durch ein Photon ausgelöste Bildung eines Thymindimers geht innerhalb von Sekundenbruchteilen vonstatten. Zwar kann ggf. eine DNA-Reparatur der geschädigten Stelle des Genmaterials stattfinden (sog. Photoreaktivierung oder Exzisionsreparatur), jedoch können im Gegensatz zum Einsatz von Desinfektionsmitteln bzw. Antibiotika keinerlei Resistenzen gegen UV-Licht gebildet werden. Das Verfahren bleibt dauerhaft wirksam.

Die Nutzung der UV-C-Strahlung zur Keimreduzierung ist eine sehr energieeffiziente und damit kostengünstige Möglichkeit der Desinfektion, welches insbesondere auf glatten Oberflächen sehr gut wirkt, wobei ein direkter Zusammenhang zwischen der Strahlendosis und der letalen Wirkung der Strahlung zu verzeichnen ist.

Ein weiterer Vorteil des Einsatzes von UV-C-Strahlung zur Keimreduzierung besteht darin, dass die eigentliche Desinfektionswirkung augenblicklich eintritt. Für eine ausreichende Keimreduzierung sind vergleichsweise kurze Bestrahlungsdauern erforderlich. Das Verfahren zur Keimreduzierung geht also vergleichsweise schnell vonstatten, ist energiesparend und chemikalienfrei.

Als zusätzlicher Vorteil ist zu benennen, dass die UV-C-Strahlung auch gegen Dauerstadien von Mikroorganismen sehr gut wirksam ist, etwa zur Inaktivierung der bereits erwähnten Kryptosporidien-Oozysten.

Konventionell werden als UV-Strahler Niederdruck-Quecksilberdampflampen eingesetzt, welche Licht in verschiedenen Bändern erzeugen, u.a. im kurzwelligen UVC-Bereich mit einem Peak bei 254 nm. Als Nachteile dieser Leuchtmittel sind zu benennen ihre Alterung und ihre relativ geringe Lebensdauer, die Gefahren einer Umweltbelastung durch Quecksilber, die lange Aufwärmzeit, ein hoher Stromverbrauch sowie die sperrigen Abmaße. Zudem ist die erreichbare Entkeimungsrate nicht optimal. Sie liegt deutlich unter 100 %. Zudem sind solche Lampen bruchgefährdet, was einem Einsatz im Tierbereich entgegensteht.

Neuere Methoden zur UV-Lichterzeugung basieren auf UVC-LED-Dioden. Diese Leuchtmittel benötigen keine Aufwärmphase, sind langlebig, sehr kompakt, nicht giftig und können mit geringen Gleichspannungen betrieben werden. All diese Eigenschaften prädestinieren diese zum Einsatz im Kontext der vorliegenden Erfindung, wo der dezentrale Einsatz der Leuchtmittel im Tierbereich an der Station präferiert wird.

Weitere Merkmale und Vorteile der Erfindung ergeben sich aus den beigefügten Ansprüchen und der nachfolgenden Beschreibung, in der mehrere Ausführungsbeispiele anhand der schematischen Zeichnungen im Einzelnen erläutert werden. Dabei zeigt:

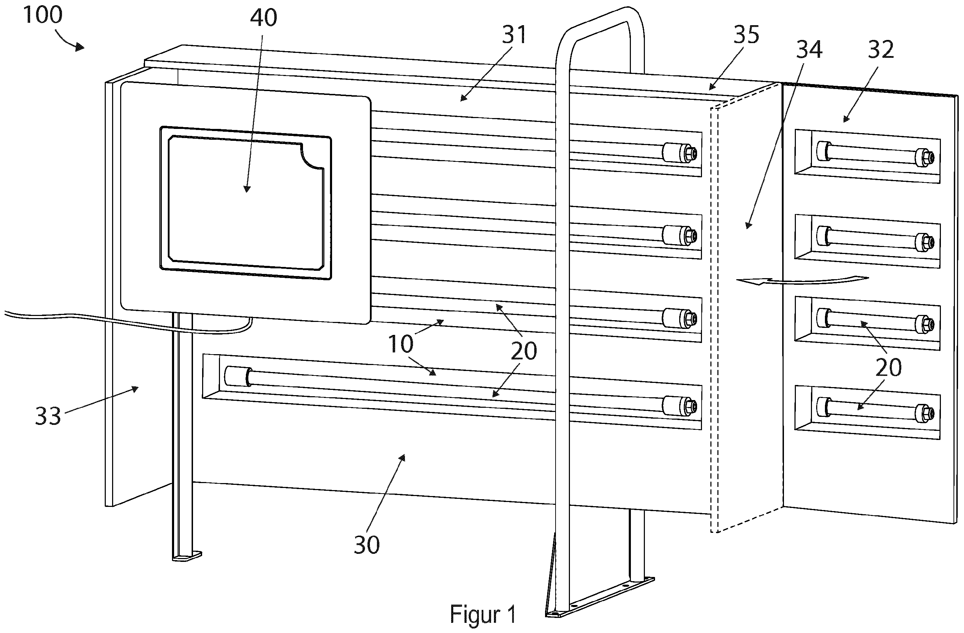

- Fig. 1: eine Behandlungsstation zur insbesondere automatisierten keimreduzierenden Bestrahlung des Integuments von Tieren, insbesondere von Jungtieren, insbesondere von Kälbern gemäß einer besonderen Ausführungsform der vorliegenden Erfindung;

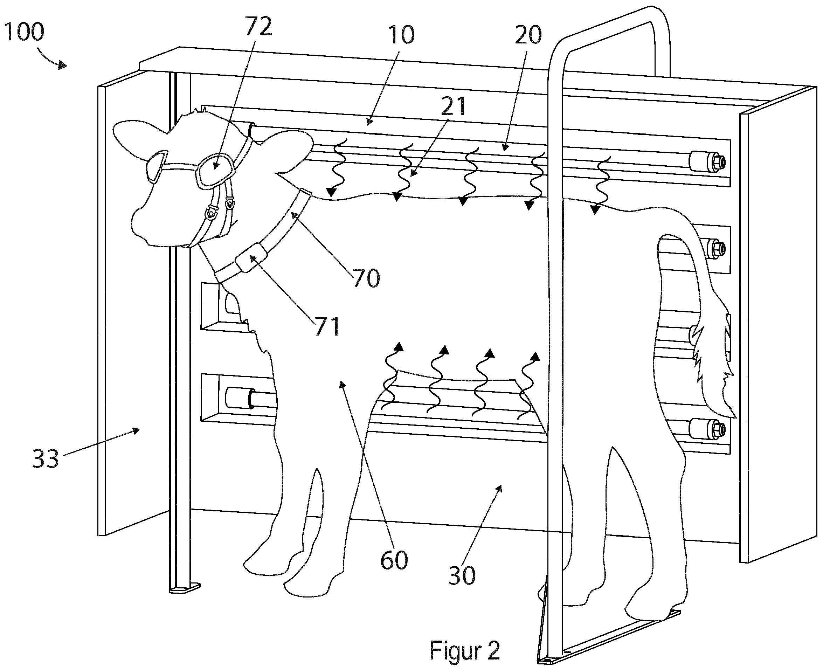

- Fig. 2: eine Behandlungsstation zur insbesondere automatisierten keimreduzierenden Bestrahlung des Integuments von Tieren, insbesondere von Jungtieren, insbesondere von Kälbern, gemäß einer weiteren besonderen Ausführungsform der vorliegenden Erfindung gemeinsam mit einem mit einer Strahlenschutzausrüstung, insbesondere zum Schutz der Augen vor UV-C-Strahlung, ausgestatteten Tier, welches aktuell einer keimreduzierenden Behandlung unterzogen wird;

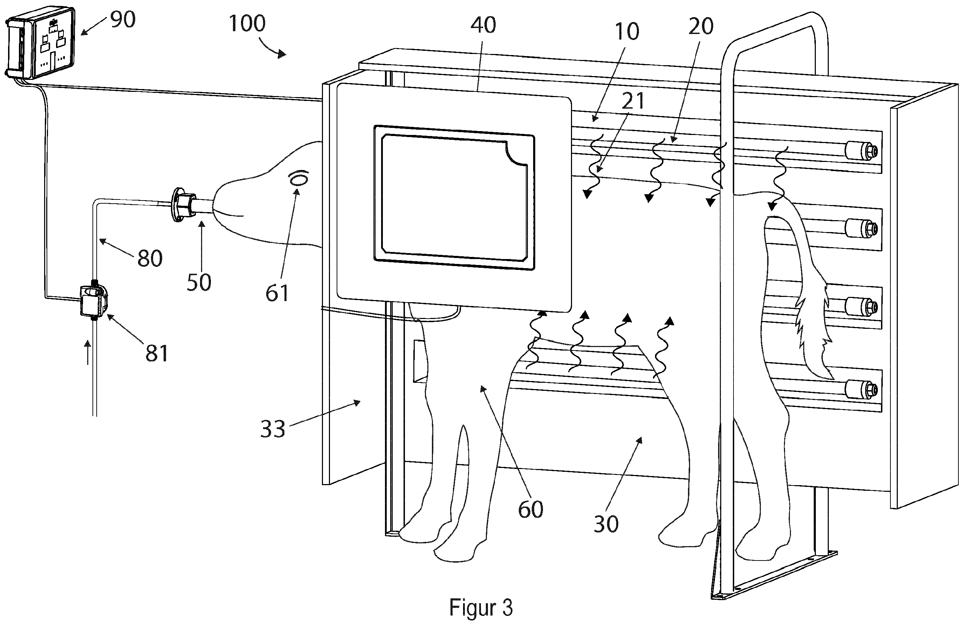

- Fig. 3: eine als eine Behandlungsstation zur insbesondere automatisierten keimreduzierenden Bestrahlung des Integuments von Tieren, insbesondere von Jungtieren, insbesondere von Kälbern, ausgebildete Tränkestation zum Entnehmen eines flüssigen Futtermittels, insbesondere Milchtränke, aus einem Fütterungsautomaten für Tiere, insbesondere für Jungtiere, z. B. Kälber gemäß einer besonderen Ausführungsform der vorliegenden Erfindung;

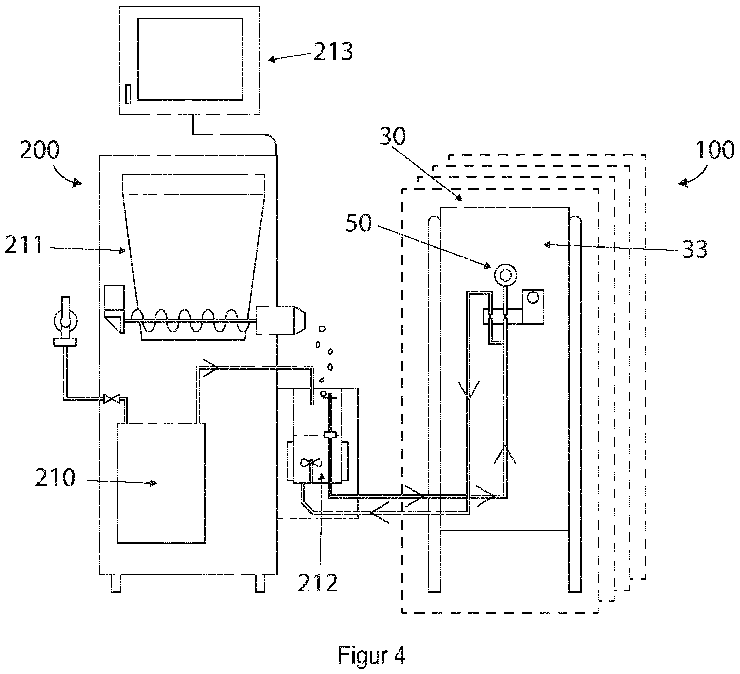

- Fig. 4: einen Fütterungsautomat mit mindestens einer als Behandlungsstation zur insbesondere automatisierten keimreduzierenden Bestrahlung des Integuments von Tieren, insbesondere von Jungtieren, insbesondere von Kälbern ausgebildeten Tränkestation zum Entnehmen eines flüssigen Futtermittels, insbesondere Milchtränke, aus dem Fütterungsautomaten für Tiere, insbesondere für Jungtiere, Kälber, gemäß einer besonderen Ausführungsform der vorliegenden Erfindung;

Figur 1 zeigt eine Behandlungsstation 100 für Tiere beispielsweise zum Entkeimen zumindest eines Teils der Fell- und Hautoberfläche (auch Integument genannt) von in der Tier-Einzelbox 30 befindlichen Tieren. Die Tier-Einzelbox 30 weist in diesem Beispiel einen offenen Zugang 34 auf. In beide Seitenwände (31, zweite Wand nicht abgebildet) sowie in die als Rückwand 32 bezeichnete Seitenwand ist je eine UV-C-Keimreduzierungseinrichtung 10 integriert. Letztere beinhalten als Wirkelement mehrere UV-C-Strahler, von denen nur einige Strahler mit der Bezugszahl 20 gekennzeichnet ist und welche in mehreren Ebenen über die gesamte Länge der Seitenwände 31 der Behandlungsstation 100 angeordnet sind, um die gesamte äußere Oberfläche des Tieres zu bestrahlen und in diesem Zug das Erbgut von auf der Fell- und Hautoberfläche befindlichen Bakterien und Keimen zu schädigen und diese damit zum Absterben zu bringen. Alternativ oder zusätzlich kann mindestens ein UV-C-Strahler an einer Decke der Behandlungsstation angebracht sein. Vorzugsweise sind geeignete Schutzeinrichtungen (nicht gezeigt) zu implementieren, um eine Zerstörung der UV-C-Strahler 20 durch z. B. Stöße von Tieren, insbesondere Jungtieren, zu vermeiden und freie Glassplitter im Stall zu verhindern. Mit diesen UV-C-Strahlern 20 können Tiere in der Behandlungsstation bspw. täglich für eine vorzugsweise kurze Zeit (ca. 30 Sek. bis ca. 240 Sek.) mit UV-C-Strahlung 21 bestrahlt werden. Diese Bestrahlung ist insbesondere für erkrankte Kälber gedacht, etwa für Tiere, die an Kälberflechte erkrankt sind, sowie für Tiere, die an Diarrhoe leiden und damit potentielle Ausscheider von hochinfektiösen Kryptosporidien-Oozysten darstellen. Im Falle von Diarrhoe ist zur Verhinderung von Schmierinfektionen insbesondere auch die Bestrahlung des Hinterteils des Tieres indiziert. Hierzu sind in diesem Beispiel in der Rückwand 32 der Behandlungsstation 100, welche verschließbar, vorzugsweise automatisiert verschließbar ausgeführt ist, ebenfalls UV-C-Strahler 20 integriert. Die Behandlungsstation 100 kann optional eine Tieridentifikationseinrichtung 40 aufweisen. In diesem Fall müssen die in der Behandlungsstation zu behandelnden Tiere wie z. B. Kälber zur Identifikation entsprechend mit elektronischen Tiertranspondern (siehe Figur 2), bspw. in der Form von RFID-Chips ausgestattet sein. Dadurch kann die tägliche Bestrahlung in der Behandlungsstation 100 automatisiert erfolgen.

Die Breite der Tier-Einzelbox 30 der Behandlungsstation 100 ist vorzugsweise kaum größer als die Tierbreite, um ein Bedrängen eines Tieres in der Behandlungsstation durch Artgenossen zu verhindern.

Die Tiere können zur Behandlung beispielsweise von einer Person in die Behandlungsstation getrieben werden.

FIG. 2 zeigt eine Behandlungsstation 100 gemäß einer weiteren besonderen Ausführungsform der Erfindung. In dieser Ausführungsform, die in weiten Teilen ähnlich wie die in Figur 1 gezeigte Ausführungsform gestaltet ist, ist die als Vorderwand 33 bezeichnete Seitenwand der Tier-Einzelbox 30 vollflächig ausgeführt, so dass sich auch der Kopf und insbesondere die Augen 61 eines Tieres 60 innerhalb der Behandlungsstation 100 befinden und ohne Schutzmaßnahmen damit der emittierten LTV-C Strahlung 21 der UV-C-Strahler 20 ausgesetzt wären. Deswegen wurde das Tier 60 zum Strahlenschutz im Kopfbereich mit einer Schutzausrüstung 72 ausgestattet. Dies soll verhindern, dass die Kopfpartie und insbesondere die Augen 61 des Tieres 60 bei der Bestrahlung Schaden nehmen. Die abgebildete Schutzausrüstung 72 des Tieres deckt im Wesentlichen die Augenpartie ab. Es können aber auch Ausführungsformen der Schutzausrüstung zum Einsatz kommen, die großflächiger ganze Teile des Gesichtsbereichs abdecken, oder es können Schutz-Kontaktlinsen zur Anwendung kommen, welche in die Augen der Tiere eingesetzt werden.

Figur 3 zeigt eine als eine Behandlungsstation 100 ausgebildete Tränkestation gemäß einer besonderen Ausführungsform der Erfindung. Hierbei ist die Tier-Einzelbox 30 der Behandlungsstation 100 als Tränkestation mit einem assoziierten Tränkenuckel 50 zum Entnehmen eines flüssigen Futtermittels, in diesem Beispiel Milchtränke, aus einem Fütterungsautomaten (nicht abgebildet) für Tiere, in diesem Beispiel Kälber, ausgeführt. Die Figur 3 zeigt ein Tier 60, welches zum Zweck der Nahrungsaufnahme die mit der Tränkestation assoziierte Tier-Einzelbox 30 aufgesucht hat und welches automatisiert mit UV-C-Strahlung 21 mittels einer UV-C-Keimreduzierungseinrichtung 10, die UV-C-Strahler 20 umfasst, behandelt wird. Um zur Nahrungsaufnahme an den Tränkenuckel 50 zu gelangen, muss das Tier 60 seinen Kopf durch eine Öffnung in der Frontwand 33 der Tränkestation stecken. Dadurch befinden sich der Kopf und insbesondere die Augen 61 des Tieres, welche vor der UV-Strahlung zu schützen sind, außerhalb des durch die Wände der Tränkestation gebildeten abgeschlossenen Raums. Der Beginn des Trinkvorgangs kann durch einen in der Saugleitung 80 verbauten Saugsensor 81 (Durchflussmesser, Unterdrucksensor) detektiert werden. Das Signal des Saugsensors 81 wird von einer Tränkestationselektronik 90 ausgewertet, welche daraufhin die Bestrahlung startet. Über den Saugsensor 81 können auch Trinkpausen oder das Ende des Trinkvorgangs erkannt werden. Die Bestrahlung wird in diesem Fall unterbrochen bzw. beendet. Diese Schutzmaßnahme erlaubt es, auf eine Schutzeinrichtung für die Augen bzw. für den Kopfbereich des Tieres 60 zu verzichten. Alle Tiere einer zu fütternden Gruppe sind in diesem Beispiel an dem mit der Tränkestation assoziierten Tränkeautomaten registriert.

Figur 4 zeigt einen Fütterungsautomaten 200 zur Zubereitung von Tränke für Jungtiere, umfassend einen Boiler oder Wärmetauscher 210 zur Erwärmung von Wasser bzw. Milch, eine Dosiereinrichtung 211 für z. B. Milchpulver und einen Anmischbehälter 212 zum Zubereiten einer Tränkeportion. Mit dem Fütterungsautomaten 200 assoziiert sind in diesem Beispiel bis zu vier als Behandlungsstationen 100 ausgebildete Tränkestationen, in denen Tiere einer keimreduzierenden Behandlung unterzogen werden können. Eine Anzeige- und Bedieneinheit 213 des Fütterungsautomaten 200 wird in diesem Beispiel genutzt, um tierindividuelle Behandlungen und Bestrahlungsdauern festzulegen, und gestattet es, den Behandlungserfolg manuell einzugeben und zu dokumentieren. Damit wird es möglich, selektiv nur bestimmte Kälber, etwa nur erkrankte Kälber, für eine Behandlung mit UV-C-Strahlung 21 auszuwählen. Des Weiteren können nach erfolgter Diagnose innerhalb des Tierbestands je nach dem Schweregrad der Erkrankung tierindividuell unterschiedliche Bestrahlungsdauern vorgegeben werden.

In der obigen Beschreibung wurden Anwendungsszenarien zur Keimreduzierung des Integuments von Nutztieren beschrieben. Erreicht wird die Keimreduzierung dabei jeweils mittels folgender Maßnahmen:
- Bestrahlung des Tieres in einer Wassertränkestation,
- Bestrahlung des Tieres in einer Futterstation, insbesondere einer Milchtränkestation, wobei in letzterem Fall die Tränkestation mit einem Fütterungsautomaten gekoppelt sein kann.

Diese Maßnahmen können einzeln oder in beliebigen Kombinationen erfolgen.

Die in der vorstehenden Beschreibung, in den Zeichnungen sowie in den Ansprüchen offenbarten Merkmale der Erfindung können sowohl einzeln als auch in den beliebigen Kombinationen für die Verwirklichung der Erfindung in ihren verschiedenen Ausführungsformen wesentlich sein.

### Bezugszeichenliste

- 10: Keimreduzierungseinrichtung
- 20: UV-C-Strahler
- 21: UV-C-Strahlung
- 30: Tier-Einzelbox
- 31: Seitenwand
- 32: Rückwand
- 33: Frontwand
- 34: Offener Zugang
- 35: Dach
- 40: Tieridentifikationseinrichtung
- 50: Tränkenuckel
- 60: Tier
- 61: Auge
- 70: Halsband
- 71: Transponder
- 72: Schutzausrüstung
- 80: Saugleitung
- 81: Saugsensor
- 100: Behandlungsstation
- 200: Fütterungsautomat
- 210: Boiler/Wärmetauscher
- 211: Dosiereinrichtung für Milchpulver
- 212: Anmischbehälter
- 213: Anzeige- und Bedieneinheit

## Patentansprüche

1. Behandlungsstation (100) zur insbesondere automatisierten keimreduzierenden Bestrahlung des Integuments von landwirtschaftlichen Nutztieren, insbesondere von Jungtieren, z. B. Kälbern, umfassend:
eine Tier-Einzelbox (30), einen Tier-Einzelunterstand oder dergleichen für den Innen- und/oder Außenbereich, umfassend mehrere Seitenwände (31, 32, 33), ein Dach (35) und einen offenen Zugang (34), und
mindestens eine UV-C-Keimreduzierungseinrichtung (10) zur keimreduzierenden Bestrahlung des Integuments von einem in der Tier-Einzelbox (30) bzw. in dem Tier-Einzelunterstand oder dergleichen befindlichen landwirtschaftlichen Nutztier, insbesondere Jungtier, z. B. Kalb.

2. Behandlungsstation (100) nach Anspruch 1, wobei die UV-C-Keimreduzierungseinrichtung (10) mindestens einen UV-C-Strahler (20) umfasst.

3. Behandlungsstation (100) nach Anspruch 1 oder 2, wobei der UV-C-Strahler (20) mittels eines Akkus und/oder einer Batterie betreibbar ist.

4. Behandlungsstation (100) nach einem der Ansprüche 1 bis 3, ferner umfassend eine Reinigungseinrichtung für den UV-C-Strahler (20).

5. Behandlungsstation (100) nach einem der vorgenannten Ansprüche, wobei die UV-C-Keimreduzierungseinrichtung (10) in mindestens einer der Seitenwände (31, 32, 33) und/oder dem Dach (30) integriert ist.

6. Behandlungsstation (100) nach einem der vorangehenden Ansprüche, ferner umfassend eine Tieridentifikationseinrichtung (40).

7. Behandlungsstation (100) nach einem der vorangehenden Ansprüche, wobei der offene Zugang (34) nach Betreten eines Tieres (60) automatisiert verschließbar ist.

8. Behandlungsstation (100) nach einem der vorangehenden Ansprüche, ferner umfassend eine Steuereinrichtung zur Einschaltung der UV-C-Keimreduzierungseinrichtung (10) für eine vorgebbare, vorzugsweise tierindividuelle, Zeitdauer beginnend bei oder nach Betreten eines Tieres (60).

9. Behandlungsstation (100) nach einem der vorangehenden Ansprüche, ferner umfassend eine Anwesenheits- oder Annäherungsdetektionseinrichtung zum Detektieren der Anwesenheit eines Tieres (60) in der Tier-Einzelbox (30) oder dem Tier-Einzelunterstand oder dergleichen oder der Annährung eines Tieres (60) an die Tier-Einzelbox (30) oder dem Tier-Einzelunterstand oder dergleichen.

10. Als eine Behandlungsstation (100) nach einem der vorangehenden Ansprüche ausgebildete Tränkestation, umfassend eine Entnahmeeinrichtung zum Entnehmen eines flüssigen Futtermittels, insbesondere Milchtränke, aus einem Fütterungsautomaten (200) oder zum Entnehmen von Tränkewasser.

11. Tränkestation nach Anspruch 10, wobei die Entnahmeeinrichtung außerhalb der Tier-Einzelbox (30) bzw. des Tier-Einzelunterstandes oder dergleichen angeordnet und durch eine Öffnung in einer der Seitenwände für ein in der Tier-Einzelbox (30) bzw. dem Tier-Einzelunterstand befindliches Tier von innen zugänglich ist.

12. Tränkestation nach Anspruch 10 oder 11, ferner umfassend eine Trinkdetektionseinrichtung, um Trinken eines in der Tränkestation befindlichen Tieres (60) detektieren zu können.

13. Tränkestation nach Anspruch 12, ferner umfassend eine Tränkestationselektronik (90), die gestaltet ist, um in Abhängigkeit von einer Detektion eines trinkenden Tieres mittels der Trinkdetektionseinrichtung die mindestens eine UV-C-Keimreduzierungseinrichtung (10) ein- und auszuschalten.

14. Fütterungsautomat (200), umfassend mindestens eine Tränkestation nach einem der Ansprüche 10 bis 13.

15. System aus einer Behandlungsstation (100) nach einem der Ansprüche 1 bis 9, einer Tränkestation nach einem der Ansprüche 10 bis 13 oder einem Fütterungsautomaten (200) und mindestens einer Schutzausrüstung (72) zum Schutz eines Tieres (60) vor UV-C-Strahlung (21), wobei die Schutzausrüstung (72) mindestens eine Schutzbrille oder Schutzkontaktlinsen umfasst.

16. Verfahren zur insbesondere automatisierten keimreduzierenden Bestrahlung des Integuments von landwirtschaftlichen Nutztieren, insbesondere von Jungtieren, z. B. von Kälbern, vorzugsweise unter Verwendung einer Behandlungsstation (100) nach einem der Ansprüche 1 bis 9 oder einer Tränkestation nach einem der Ansprüche 10 bis 14 oder eines Systems nach Anspruch 15, umfassend die Schritte:
- Automatisierte Identifikation eines Tieres, Ermittlung der Notwendigkeit einer Behandlung des Tieres (60) mit UV-C-Strahlung (21) und Abruf einer vorab tierindividuell festgelegten Behandlungsdauer anhand eines Tierkennungs-Chips in der Behandlungsstation (100) oder Tränkestation und
- Behandlung des Tieres (60) mit UV-C-Strahlung (21) mit besagter Behandlungsdauer.

17. Verfahren nach Anspruch 16, ferner umfassend Dokumentation eines Bestrahlungsergebnisses.
